# EUROPEAN PATENT SPECIFICATION

(11) **EP 3 481 468 B1**
(45) Date of publication and mention of the grant of the patent: **13.05.2020**
(21) Application number: 17740704.6
(22) Date of filing: 07.07.2017
(51) Int. Cl.: A61M 5/31, A61M 5/315

(54) **DRUG DELIVERY DEVICE COMPRISING AN ADJUSTMENT MEMBER**
WIRKSTOFFFREISETZUNGSVORRICHTUNG MIT ANPASSUNGSELEMENT
DISPOSITIF D'ADMINISTRATION DE MÉDICAMENT COMPRENANT UN ÉLÉMENT DE RÉGLAGE

(30) Priority: 08.07.2016 EP 16178694
(43) Date of publication of application: 15.05.2019
(73) Proprietor: Novo Nordisk A/S, 2880 Bagsværd (DK)
(72) Inventor: BENGTSSON, Henrik, 2800 Bagsværd (DK); SPORK, Emil, Gram, 2200 Copenhagen N (DK)
(86) International application number: PCT/EP2017/067153
(87) International publication number: WO 2018/007623

(56) References cited:
- WO-A1-2010/124961
- WO-A1-2011/121061
- US-A1- 2009 054 846

## Description

The present invention relates to an assembly of components for a drug delivery device that allows a user to select single or multiple doses of an injectable liquid drug and to dispense the set dose of the product and to apply said product to a patient, preferably by injection. In particular, the present invention relates to a configuration that enables optimal assembling of such drug delivery devices.

### BACKGROUND

In the disclosure of the present invention reference is mostly made to drug delivery devices used e.g. in the treatment of diabetes by delivery of insulin, however, this is only an exemplary use of the present invention.

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe. Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the liquid drug to be administered is provided in a cartridge that has a moveable piston or bung mechanically interacting with a piston rod of an expelling mechanism of the drug delivery device. By applying thrust to the piston in distal direction, a predefined amount of the liquid drug is expelled from the cartridge. Due to inevitable manufacturing tolerances there may for instance persist axial clearance between a cartridge's piston and the piston rod. Typically, prior to a primary use of the device, an end-user has to conduct a so-called priming of the expelling mechanism in order to ensure, that already with an initial dose setting and a first subsequent dose dispensing step, an accurate amount of the liquid drug is dispensed in a predefined way. An initial dose setting and expelling of a minor dose may in certain situations also be required for removing any air present in the cartridge and/or a connected needle and for performing a flow check.

Document WO 99/38554 A1 discloses several embodiments of injection devices each suitable for forming a disposable device wherein a liquid drug cartridge is inserted into the device during assembly in a production line.

State of the art pen-type drug delivery devices that incorporate a dose setting feature often include a so-called end-of-content limiter to prevent a user from selecting a size of a dose which exceeds the amount of liquid drug remaining in a cartridge of the device. References WO 01/19434 A1, WO 2006/128794 A2, WO 2010/149209 A1 and WO 2013/156224 A1 include disclosure of such end-of-content limiters.

In the production line, during final assembly operations of the devices, at least a part of the priming is typically carried out using the dose setting and expelling mechanism so that users will experience virtually consistent requirement for a priming operation across individual pen samples irrespective of the initial gap between the piston rod and the piston which emanates from tolerance variations. Reference WO 2009/095332 A1 discloses devices wherein a distance between the distal end of a piston rod means and the plunger is minimized or reduced to zero. WO 2010/124961 A1 and WO 2011/121061 A1 provides further disclosure of related methods and devices.

### SUMMARY

It is an object of the present invention to provide a drug delivery device featuring improved and facilitated clearance reduction or clearance elimination. It is a further object to provide a simplified and robust method of eliminating clearance in a drug delivery device. Finally, it is an object to provide manufacture of drug delivery devices providing consistently uniform and predictable total doseable amount of liquid drug from a held cartridge, aiming in reducing requirements with regard to performing control measurements during manufacturing.

In the disclosure of the present invention, embodiments and aspects will be described which will address one or more of the above objects or which will address objects apparent from the below disclosure as well as from the description of exemplary embodiments.

In a first aspect, the present invention relates to a drug delivery device for dispensing one or more doses of a medicament, comprising:
- a medicament cartridge, wherein the cartridge comprises (i) a distal end, (ii) a proximal end that is opposite the distal end along a body axis of the cartridge, and (iii) a movable piston arranged substantially at the proximate end of the cartridge,
- a drive mechanism comprising a piston rod configured for axially moving the piston in a distal direction,
wherein the piston rod connects to an adjustment member located at least partially between the piston rod and a proximal face of the piston, wherein the adjustment member is configured to abut or cooperate with the proximal face of the piston to reduce an axial clearance between the piston rod and the proximal face of the piston,
wherein one of the piston rod and the adjustment member comprises a first contour system comprising a plurality of axially facing steps being circumferentially disposed and arranged axially offset from one another,
wherein the other of the piston rod and the adjustment member comprises at least one radially disposed protrusion,
wherein said at least one radially disposed protrusion axially engages a selective one of said plurality of axially facing steps of the first contour system so that the relative rotational position between the piston rod and the adjustment member determines the axial position of the adjustment member relative to the piston rod when the adjustment member is situated in full axial engagement with the piston rod, and
wherein the piston rod comprises a first rotational block geometry and the adjustment member comprises a second rotational block geometry to cooperate with the first rotational block geometry to prevent the said at least one radially disposed protrusion from disengaging the selected one of said plurality of axially facing steps of the first contour system when the adjustment member is situated in full axial engagement with the piston rod.

In accordance with the first aspect the invention provides a simple yet effective means of adjusting or eliminating clearance between the piston rod and the piston of a held cartridge during assembly operations. By a simple adjustment procedure, the axial position between the adjustment member and the piston is adjusted and thus an initial clearance may be reduced into a predefined magnitude or, alternatively, eliminated completely. The simple adjustment procedure provides for a predictable clearance compensation which will require little or no control measurements.

In some embodiments the said at least one radially disposed protrusion defines a plurality of radially disposed protrusions. The plurality of radially disposed protrusions may be configured to define a second contour system wherein the plurality of radially disposed protrusions are arranged axially offset to one another to cooperate and engage with respective axially facing steps of the first contour system.

In some embodiments, each said plurality of axially facing steps defines a bottom surface of a respective axially extending channel. In such configuration, the axially extending channel may be formed as individual separate channels separated by rib geometries such that once a protrusion has been received by a selected channel the protrusion cannot be moved into a neighbouring channel.

In further embodiments each of the at least one radially disposed protrusion defines an abutment surface having a shape mating with an abutment surface defined by each of the plurality of axially facing steps.

Each of said plurality of steps of the first contour system may comprise a surface which extends substantially perpendicularly relative to the axis.

In some embodiments, when the at least one radially disposed protrusion engages a respective one of said series of axially facing steps of the first contour system, a press-fit engagement between the piston rod and the adjustment member is provided.

In some embodiments one of the piston rod and the adjustment member defines an axially extending shaft member being received in a axial bore of the other of the piston rod and the adjustment member. Said other of the piston rod and the adjustment member may further define a longitudinal member comprising the longitudinal bore in an end section thereof so that an end wall portion of the longitudinal member encircles the bore and wherein the end wall portion comprises one or more axially extending slots providing radial resiliency of annular sections divided by the one or more axially extending slots.

In some embodiments, said axial bore is defined by an end portion having walls at least partly circumscribing the axial bore. The walls circumscribing the axial bore may be configured to provide radial resiliency for retaining said axially extending shaft member when it has been fully inserted into the axial bore.

In some embodiments the adjustment member defines said at least one radially disposed protrusion. The adjustment member defines a first end and an opposite second end.

In some embodiments the adjustment member is configured for being inserted between the piston and the piston rod selectively with the first end of the adjustment member facing the piston rod or with the second end of the adjustment member facing the piston rod.

Respective ones of said at least one radially disposed protrusion are arranged at an axial distance from the first end of the adjustment member thereby defining a first set of respective distances and wherein respective ones of said at least one radially disposed protrusion are arranged at an axial distance from the second end of the adjustment member thereby defining a second set of respective distances and wherein the first set of respective distances differ from the second set of respective distances. In this way, the orientation of the adjustment member pointing with its first end either in the proximal direction or in the distal direction is decisive for the axial position of the adjustment member relative to the piston rod.

In certain embodiments, a piston washer is arranged between the adjustment member and the piston. Both the first end and/or the second end of the adjustment member may include a rotation symmetrical portion configured for mating with a bearing surface of a piston washer.

In some embodiments the drug delivery device forms a disposable device wherein the cartridge mounted within the device cannot be replaced with a new cartridge. In other embodiments, the drug delivery device forms a durable device wherein an exhausted cartridge is to be replaced by a new filled cartridge.

In some embodiments, the plurality of steps and the at least one radially disposed protrusion provide axial variation within a range of axial positions wherein said range is less than 4 % of the total stroke length that the drive mechanism is operable for moving the piston rod for expelling the entire expellable contents of the cartridge. In certain embodiments said range of axial variations is configured to provide less than 0.9 mm axial variation.

The drive mechanism includes a dose expelling mechanism. In some embodiments, the drive mechanism further includes a dose setting mechanism operable to set the size of a dose intended for subsequently being expelled from the drug delivery device. In some embodiments the drive mechanism forms a multi-dose expelling mechanism for administering a series of separate doses by injection. In still other embodiments, the drive mechanism is configured for administering only fixed sized doses, and optionally, only a single fixed sized dose.

In some embodiments, the piston rod of the drive mechanism is configured to rotate during dose expelling. In one embodiment, the piston washer is rotatably arranged relative to the piston rod to allow the piston rod to rotate while the piston washer remains non-rotatable. In some embodiments, the piston rod is provided with at least one thread along the length of the piston rod to induce an axial movement of the piston rod as the piston rod is rotated by the drive mechanism.

The drug delivery device may define a housing component that houses the drive mechanism. In some embodiments, the drive mechanism may include a driver adapted to rotationally couple to the piston rod so as to prevent relative rotational movement but allow relative axial slideable engagement between the driver and the piston rod. The driver will in such embodiments be configured to rotate during a dose expelling procedure to induce rotation on the piston rod to rotate the piston rod through a threaded nut element associated with the housing component thereby causing a dose of drug to be expelled. The nut element may comprise a threaded opening engaging the at least one thread of the piston rod.

In some embodiments, the dose setting and expelling mechanism may comprise a dose setting element that rotates relative to a driver during setting of a dose and wherein the dose setting element and the driver rotates together during expelling of a set dose.

In further embodiments of the drug delivery device according to the first aspect, the device comprises an end-of-content limiter which prevents setting a dose which exceeds a doseable amount of liquid drug remaining in the cartridge.

The end-of-content limiter may in some embodiments be arranged between the driver and the dose setting element of the drug delivery device. In exemplary embodiments the end-of-content limiter is engaging the driver and the dose setting element. In such embodiment, as the dose setting element is rotated relative to the driver for dialling up a dose, the end-of-content limiter moves towards an end-of-content stop geometry for example provided by one of the driver and the dose setting element. In other embodiments, the end-of-content limiter is engaging the piston rod and a dose setting element. In such embodiment, as the dose setting element is rotated relative to the piston rod for dialling up a dose, the end-of-content limiter moves towards an end-of-content stop geometry for example provided by one of the piston rod and the dose setting element.

In situations where and end-of-content mechanism is incorporated into the device, the manufacturing and assembly can be carried out without use of the dose setting and expelling mechanism for evening out an initial gap between the piston rod and the piston and thus further enables the end-of-content mechanism of each individual device to provide a consistent and uniform total doseable quantity from each individual pen device.

In all of the embodiments according to the invention a piston washer may be arranged between the adjustment member and the piston of a held cartridge to distribute axial forces exerted by the piston rod onto a large surface portion of the piston. In the context of the present application, when cooperation between the adjustment member and the piston is dealt with, such as by using terms such as contact, abutment, engagement or cooperation, this does not mean that a direct cooperation is provided, but may involve a an element such as a piston washer being arranged between the adjustment member and the piston, either provided as a separate entity or provided as part of the piston or the adjustment member. For example, in the context of the present disclosure, when it is defined that the adjustment member engages or abuts the piston, embodiments wherein a piston washer is arranged between the adjustment member and the piston are encompassed as well.

As used herein, the term "insulin" is meant to encompass any drug-containing flowable medicine capable of being passed through a delivery means such as a cannula or hollow needle in a controlled manner, such as a liquid, solution, gel or fine suspension, and which has a blood glucose controlling effect, e.g. human insulin and analogues thereof as well as non-insulins such as GLP-1 and analogues thereof. In the description of exemplary embodiments reference will be made to the use of insulin.

### BRIEF DESCRIPTION OF DRAWINGS

In the following the invention will be further described with reference to the drawings, wherein
fig. 1 shows a prior art injection device in an exploded view showing the various components,
fig. 2 shows schematically a prior art end-of-content limiter suitable for incorporation in an injection device similar to the one shown in fig. 1,
fig. 3 shows an end view, a side view and a perspective view of an adjustment member in a first embodiment in accordance with the first aspect of the invention,
fig. 4 shows an end view and a partial perspective view of a piston rod of the first embodiment in accordance with the invention,
fig. 5 shows a partial perspective view of and assembly of the piston rod of fig. 4 with an adjustment member of fig. 3 in a first configuration,
fig. 6 shows end views and side views of the assembly of fig. 5 respectively in the first and a second configuration,
fig. 7 shows an end view, a side view and a perspective view of an adjustment member in a second embodiment in accordance with the invention,
fig. 8 shows a partial perspective view of a piston rod of the second embodiment in accordance with the invention,
fig. 9 shows end views and side views respectively of the piston rod of fig. 8 and assemblies of the parts shown in figs. 7 and 8 in a first, a second and a third configuration,
fig. 10 shows an end view, a side view and a perspective view of an adjustment member in a third embodiment in accordance with the invention,
fig. 11 shows a partial perspective view of a piston rod of the third embodiment in accordance with the invention,
fig. 12 shows end views and side views of assemblies of the parts shown in figs.10 and 11 in a first, a second and a third configuration,
fig. 13 shows a perspective view of an adjustment member of a fourth embodiment not according to the invention,
fig. 14 left view shows a partial perspective view of a piston rod of the fourth embodiment not according to the invention whereas the right view shows the piston rod and the adjustment member of fig. 13 in an assembly,
fig. 15 is a cross-sectional side view of the parts shown in fig. 14, right view, and
fig. 16 shows a side view of a further fifth embodiment of an assembly not according to the invention.

Generally, in the figures, like structures are mainly identified by like reference numerals.

### DESCRIPTION

The shown figures are schematic representations for which reason the configuration of the different structures as well as their relative dimensions are intended to serve illustrative purposes only. In the following, when the term member or element is used for a given component, it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

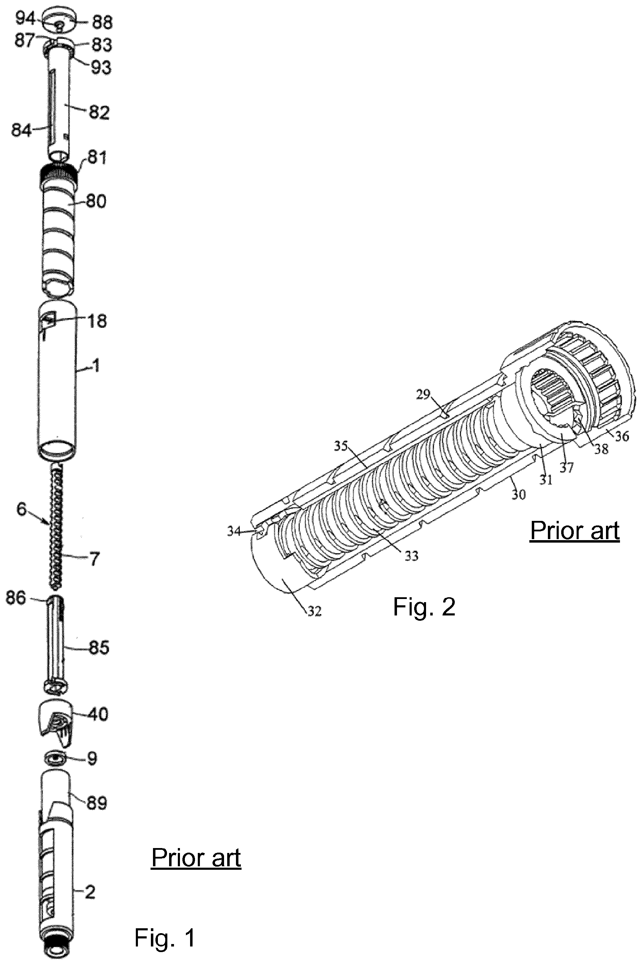

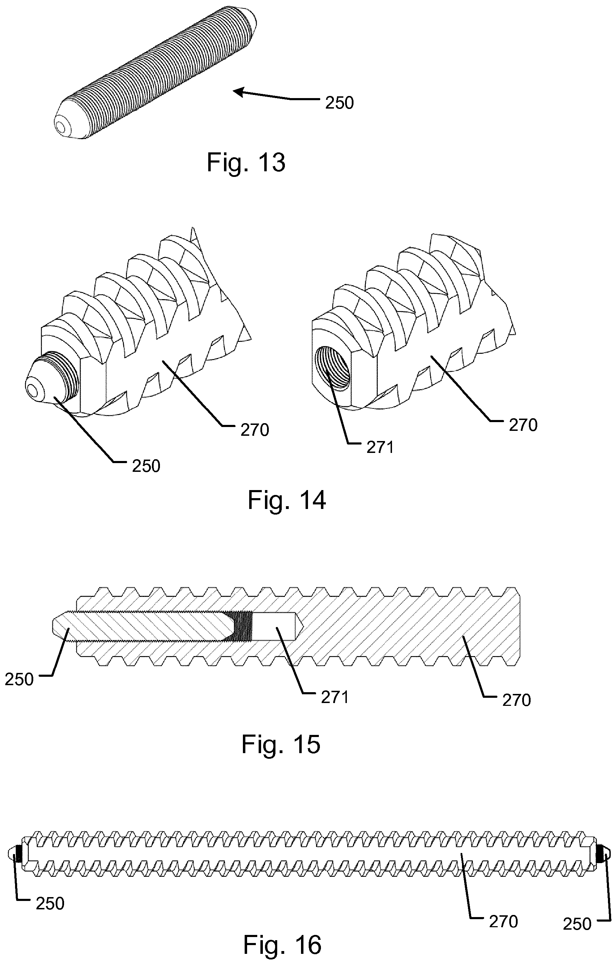

Shown in fig. 1 is an exploded view of components of a prior art pen-formed drug delivery device as disclosed in connection with fig. 15-17 of reference WO 99/38554 A1. Fig. 1 of the present disclosure is a reproduction of fig. 17 of that reference. In the present context the device represents a "generic" prior art drug delivery device providing an example of an injection pen, e.g. a pen shaped injection device which may define a central longitudinal axis along which a piston of a held cartridge is arranged for slideable movement. The injection device is provided with a drive mechanism which incorporates a dose setting and expelling mechanism for setting and expelling one or more doses of a drug, e.g. operable for selecting the size of a dose in a dose setting procedure and expelling the set dose during a dose expelling procedure. A drug delivery device closely related to the device shown in fig. 1 is marketed by Novo Nordisk as NovoPen FlexPen®.

More specifically, the pen device shown in fig. 1 comprises a cap part (not shown) and a main part having a proximal body or drive assembly portion with a proximal housing component 1 in which a drug expelling mechanism is arranged or integrated, and a distal housing component 2 forming a cartridge holder portion in which a drug-filled transparent cartridge 89 (with a non-referenced distal needle-penetrable septum) can be arranged and retained in place. The distal end of the distal housing component 2 thus forms a drug outlet.

Only components directly necessary for the understanding of the present disclosure will be described herein. For the full description of the expelling mechanism reference is made to WO 99/38554 A1 which is hereby incorporated by reference.

In the fully assembled state, the distal housing component 2 is fixedly attached to the proximal housing component 1, e.g. by an axial snap connection, the distal housing component 2 having openings allowing a portion of the cartridge 89 to be inspected. The cartridge may for example contain an insulin, GLP-1 or growth hormone formulation. The device is designed to irreplaceably accommodate a cartridge 89 inserted through a proximal receiving opening in the distal housing component 2, the cartridge being provided with a piston driveable by a piston rod forming part of the expelling mechanism. A piston washer 9 may be located between the piston rod and the piston for transferring axial forces exerted by the piston rod to the piston.

Again referring to fig. 1, a nut element 40 is fixedly secured to the distal housing component 2 at a predefined axial location thereof. The nut element 40 defines a central opening extending a along a central axis of the injection device, the opening defining an inner thread. A piston rod 7 includes an outer thread adapted to engage the inner thread of the nut element 40. The piston rod 7 further includes a longitudinal recessed track 6, a pair of flattened surfaces or similar longitudinal extending geometry for cooperating with a mating geometry of a driver tube 85 of the expelling mechanism to enable relative axial movement between the piston rod 7 and the driver tube 85 but prevent relative rotation. In the shown embodiment, the driver tube 85 is part of a multi-piece driver comprising driver tube 85 and bushing 82. Driver tube 85 and bushing 82 are configured for rotating together but to be axially displaceable relative to each other. Driver tube 85 is mounted for rotation at an axially fixed location with respect to proximal housing component 1. Driver tube 85 is rotated during dose expelling but remains non-rotatable during dose setting. A dose setting element formed as a scale drum 80 is mounted in a thread of the proximal housing component 1 and arranged to encircle and axially overlap bushing 82. During dose setting the scale drum 80 is dialled up by rotating in the thread of proximal housing component 1. Scale drum 80 is thus moved in a proximal direction relative to the proximal housing component 1 to extend proximally a distance proportional to the size of the set dose. During this dose setting procedure, the bushing 82 is moved along axially but bushing is prevented from rotating relative to the housing. A set dose is expelled by a user forcing an injection button 88 in the distal direction. During this, the scale drum 80 is rotated back towards an initial position. The bushing 82 is coupled to the scale drum 80 so that it rotates with the scale drum 80 during injection. The driver tube 85 is forced to rotate as well and acts to rotate the piston rod 7 through the nut element 40. In accordance herewith the piston rod 7 transfers a force in the distal direction forcing the piston of the cartridge 89 to move distally for expelling a quantity equal to size of the set dose.

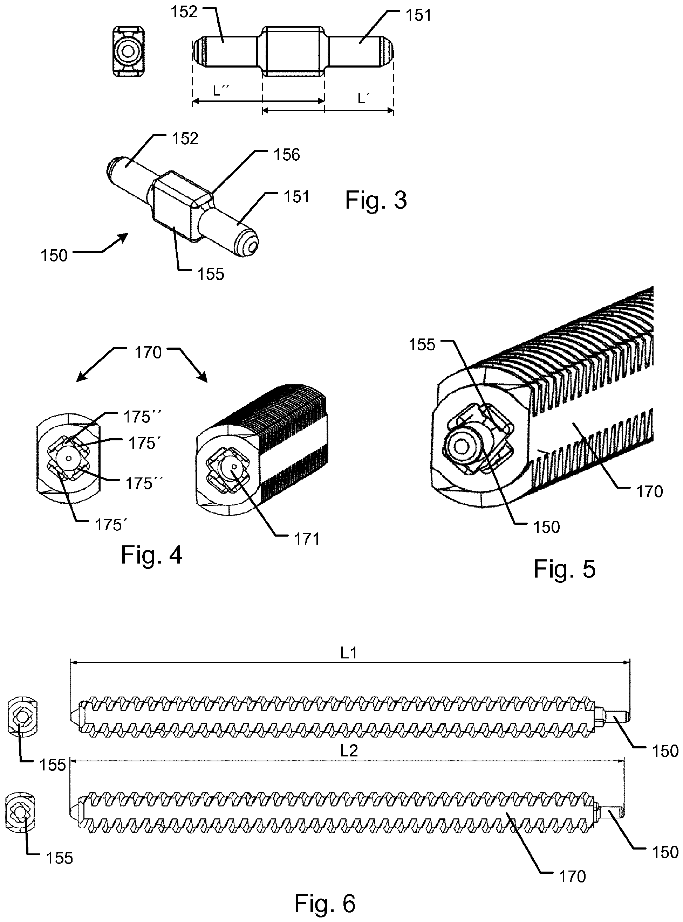

Particular variants of the injection pen shown in fig. 1 may include a dose setting limiter which prevents setting of a dose exceeding the doseable amount of liquid drug remaining in the cartridge of the injection device. One suitable dose setting limiter, i.e. a so-called "end-of-content limiter" is disclosed and shown in fig. 3 of WO 01/19434 A1, and further reproduced in the present disclosure in fig. 2. In the embodiment shown, the driver 31 forms an element generally corresponding to the driver of the device shown in fig. 1, i.e. bushing 82. An outer helical track 33 is disposed on the driver 31. A dose setting limiter is defined by nut member 32 which is threadedly coupled to helical track 33 of driver 31. Further, in fig. 2, a dose setting element 30 corresponds to the scale drum 80 of fig. 1. At its outer wall the nut member 32 is in the axial direction provided with a recess 34 which is engaged by a ridge 35 in the axial direction on the inner side of the dose setting element 30.

The helical track has a length which correlates with the total amount of drug in a new full cartridge. During dose setting the driver 31 remains non-rotatable while the dose setting element 30 rotates. Hence, when dialling up a dose the nut member 32 moves towards an end stop of helical track 33. During dose expelling the driver 31 rotates with the dose setting element 30. Hence nut member 32 maintains its present position relative to helical track 33. The nut member 32 only abuts the end stop of the helical thread 33 when the sum of accumulated expelled doses and the present set dose equals the total amount of liquid drug in a full cartridge. This prevents setting a dose that exceeds the amount of liquid drug remaining in the cartridge.

The shown end-of-content limiter provides only a non-limiting example of a suitable end-of-content limiter to be used in connection with the injection device shown in fig. 1. Other known end-of-content limiters may alternatively be used in the injection device, such as the end-of-content limiters disclosed in WO 2006/128794 A2, WO 2010/149209 A1 and WO 2013/156224 A1. In accordance with such end-of content mechanisms, instead of an end-of-content limiter formed as a nut member as such, a corresponding track follower that does not necessarily be formed as a "nut" may be used instead, where the track follower may include a first track coupling being coupled to a driver and a second track coupling being coupled to a dose setting element. When used in the present disclosure, the term "nut member" and "end-of-content limiter" will encompass any type of end-of-content limiter.

In accordance with the assembling procedure of the prior art injection device shown in fig. 1, the device may be assembled by forming a distal subassembly which includes distal housing component 2, cartridge 89, piston washer 9, nut element 40 and piston rod 7. In distal subassembly, the nut element 40 is secured relative to the distal housing component 2 at a particular predefined axial position by means of an axial snap fit. The piston rod 7 is provided in an already threadedly engaged state relative to nut element 40 where the piston rod 7 assumes a particular rotational orientation relative to distal housing component 2 and where the distal end of the piston rod is in close proximity with the piston washer 9, the piston washer being located adjacent the piston of cartridge 89.

The proximal subassembly includes the remaining components of the injection device, i.e. the proximal subassembly includes the proximal housing component 1 and the dose setting and expelling mechanism in a state where the nut member 32 assumes a predefined start position relative to the helical track of the driver (i.e. bushing 82) and where the driver tube 85 assumes a predefined rotational orientation relative to the proximal housing component 1. This allows the piston rod 7 to slide into engagement with the driver tube 85 as the distal subassembly is axially brought into engagement with the proximal subassembly for finally securing the two subassemblies relative to each other, this without requiring relative rotation between piston rod 7 and driver tube 85.

Production tolerances on the piston rod, the dose setting and expelling mechanism, cartridge body, cartridge filling level and other components result in variations in piston rod position and piston position in each device during assembly.

In mechanical devices production, in order to minimize a potential clearance between the piston rod and the piston of the cartridge, positioning may be carried out by initially positioning the piston rod 7 in a nominal position. Due to tolerances various different clearance gaps between the piston and the piston rod will show when the distal and proximal subassemblies of each sample are permanently secured together. On the basis of measurements or estimations, which may be performed at different steps of the assembly process, the actual gap in each sample traditionally has been eliminated or at least partly reduced by operating the dose setting and expelling mechanism. Operating the dose setting and expelling mechanism may be carried out either after final assembly or prior to final assembly of the different subassemblies. However such compensation procedure means that the end-of-content mechanism will be operated to a lesser or higher degree even before the device is shipped to the user meaning that the experienced total doseable volume varies from sample to sample. Generally such variations and inconsistencies from one sample to another should be avoided as this may provide the impression that the quality of the device could be somewhat non-optimal.

Turning now to figs. 3 and 4 a first embodiment of an improved drug delivery device and assembly method will now be described which generally may be of the same overall design as the devices discussed in relation to figs. 1 and 2. Fig. 3 shows an end view, a side view and a perspective view of an adjustment member 150 to cooperate with a modified piston rod 170 as shown in fig. 4. In the first embodiment, the adjustment member 150 is formed as an elongated member extending along a central longitudinal axis and defining a first shaft formed portion at a first end 151 and a second shaft formed portion at the second opposite end 152. At a particular axial location of the adjustment member a protrusion 155 is disposed where the protrusion has radially outwards extending geometries relative to the shaft portions 151 and 152 and, hence, provides axial facing blocking surfaces on either side of the protrusion 155. The protrusion may be formed to exhibit a multifaceted outer surface with edges running axially parallel to the central axis. In the shown embodiment the protrusion 155 is formed generally rectangular cuboid to provide four edges running axially parallel to the central axis wherein each edge ends in a corner feature 156. The protrusion 155 is located between the first end and the second end. The protrusion 155 thus exhibits a first set of axial blocking surfaces pointing towards the first end 151 of the adjustment member where the first set of end surfaces are located a distance L" from the second end 152. The protrusion 155 further exhibits a second set of axial blocking surfaces pointing towards the second end 152 of the adjustment member where the second set of axial blocking surfaces are located a distance L' from the first end 151. In some embodiments the distance L' is formed with same magnitude as L". In other embodiments, the distance L' is different from L".

Fig 4 shows an end view and a partial perspective view of the distal end of a piston rod 170. In the shown embodiment the distal end face of piston rod 170 is formed with a bore 171 which is formed to receive one of the shaft formed portions of the adjustment member 150. The bore further includes a series of pockets that are angularly disposed around the central axis and each being configured to receive the edges of protrusion 155 so that each pocket is configured for receiving an edge of the protrusion 155. Each pocket forms a distally facing surface 175'/175" adapted to mate with the axial blocking surface of the protrusion 155. By angularly aligning the edges of the protrusion 155 within a selected set of pockets in the piston rod 170 the adjustment member 150 may be inserted axially into the bore 171 until further axial insertion is blocked by one or more of the axial blocking surfaces abutting distal facing surfaces of a corresponding step or set of steps 175'/175" formed in the selected set of pockets. In this position the edges of the protrusion 155 of adjustment member 150 prevents rotation of the adjustment member relative to the piston rod 170 so that the relative position of the adjustment member relative to the selected pocket cannot be changed by a relative rotational movement. Hence, the piston rod 170 comprises a first rotational block geometry and the adjustment member 150 comprises a second rotational block geometry that cooperate with each other once axial engagement has been established to prevent relative rotational movement that would otherwise potentially cause disengagement between the protrusion and it's selected pocket.

In the shown embodiment, the piston rod comprises a first contour system comprising a plurality of steps 175' and 175" wherein the steps are circumferentially disposed and arranged axially offset from one another. In the shown embodiment, a pair of first steps 175' is arranged 180 degrees apart with the first steps being disposed at the same distance from the distal end of the piston rod 170. Also a pair of second steps 175" is arranged 180 degrees relative to each other but rotated approximately at right angles with respect to the first pair of steps 175'. Both second steps 175" are disposed at the same distance from the distal end of the piston rod 170 but at a different depth relative to the first pair of steps 175'. In the shown embodiment, the first steps 175' are arranged at a more shallow depth than the second steps 175".

By inserting the first end 151 of the adjustment member 150 into bore 171 of the piston rod 170, while selecting the angular orientation of the adjustment member 150 relative to the piston rod 170, the protrusion 155 of adjustment member 150 may be introduced into a selected set of pockets in bore 171 leading to the first pair of steps 175', this situation being shown in fig. 5. This situation is also depicted in the top view of fig. 6. When the adjustment member 150 is situated in full axial engagement with the piston rod 170 the second end 152 of adjustment member protrudes a predetermined distance from the distal end face of piston rod 170. Thus the assembly formed by the piston rod and the adjustment member assumes an effective length of dimension L1. Alternatively, the protrusion 155 of adjustment member 150 may be introduced into a selected set of pockets in bore 171 leading to the second pair of steps 175". When the adjustment member 150 is situated in full axial engagement with the piston rod 170, as shown in the bottom view of fig. 6, the second end 152 of the adjustment member 150 instead protrudes a predetermined distance from the distal end face of piston rod 170 that is different from the first situation. Thus, in the shown embodiment, the assembly formed by the piston rod and the adjustment member assumes an effective length of dimension L2 which is shorter than L1.

During assembly of the drug delivery device, prior to final assembly, the axial clearance between the piston rod 170 and the piston of a held cartridge 89 can be measured or otherwise estimated. Non-limiting examples of such clearance determination may involve measuring the position of the piston relative to the cartridge or, if the cartridge is held by a housing part of the device, measuring the position of the piston relative to the housing part. And it may involve measuring the position of the piston rod with respect to a housing part of the device which accommodates the drive mechanism. In accordance with the determined axial clearance, the angular orientation between the adjustment member and the piston rod can be selected so that the effective length of the assembly formed by the piston rod and the adjustment member effectively compensates for tolerance variations in a manner that, when the adjustment member is in full axial engagement with the piston rod, the clearance between the adjustment member/piston rod and the piston is eliminated or, alternatively, reduced to a gap of a predefined axial size.

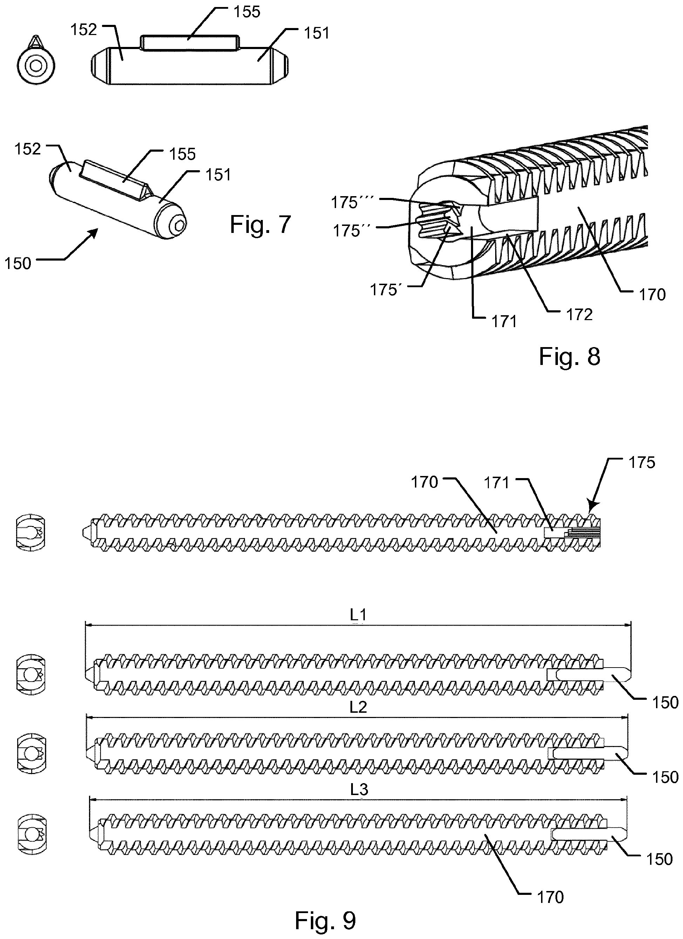

Fig. 7 and 8 show a second embodiment of components for forming an assembly of the adjustment member 150 and piston rod 170. Fig. 7 shows an end view, a side view and a perspective view of an adjustment member 150 to cooperate with a modified piston rod 170 as shown in fig. 8. In the second embodiment, the adjustment member 150 is formed as a generally cylindrical elongated member having a first end 151 and a second end 152 and with a single radial extending protrusion formed as an axially extending rib 155. The axially extending rib 155 thus forms axial blocking surfaces at either end of the rib.

Fig. 8 shows a partial perspective view of the distal end of the piston rod 170 of the second embodiment. In the shown embodiment the distal end face of piston rod 170 is formed with a bore 171 which is formed to receive an end portion of the adjustment member 150. The bore 171 further includes a series of pockets disposed circumferentially relative to each other and emerging at the distal end face of the piston rod 170. Each pocket is formed as an axial track sized to receive the radial extending protrusion 155 of adjustment member 150. In the shown embodiment, three pockets are formed, each pocket extending in the proximal direction to a respective step 175', 175" and 175''' where each step provides a distally faced surface arranged axially offset relative to the other steps. In the second embodiment, the number of pockets is selected as three. However, in other embodiments fewer or more pockets can be provided to enable tolerance compensation in accordance with a desired variability range of axial positions and in accordance with the desired resolution.

In the embodiment shown in fig. 8, the distal end of the piston rod 170 is formed with an axial slot 172 that leads radially to the bore 171. This provides flexure of the circumferential sections that act to grip the adjustment member 150 when it has been inserted into the selected pocket.

The steps 175', 175" and 175''' forms a contour system 175 of steps that are circumferentially disposed and arranged axially offset from one another. This is further visible in the upper view of fig. 9.

The three lower views of fig. 9 illustrate the variability of the length of the assembly formed by the piston rod and the adjustment member. By choosing a respective one of steps 175', 175" and 175''' to cooperate with the axial blocking surface of the radially extending protrusion 155 of adjustment member 150 the length of the assembly formed by the piston rod and the adjustment member can be chosen as L1, L2 or L3.

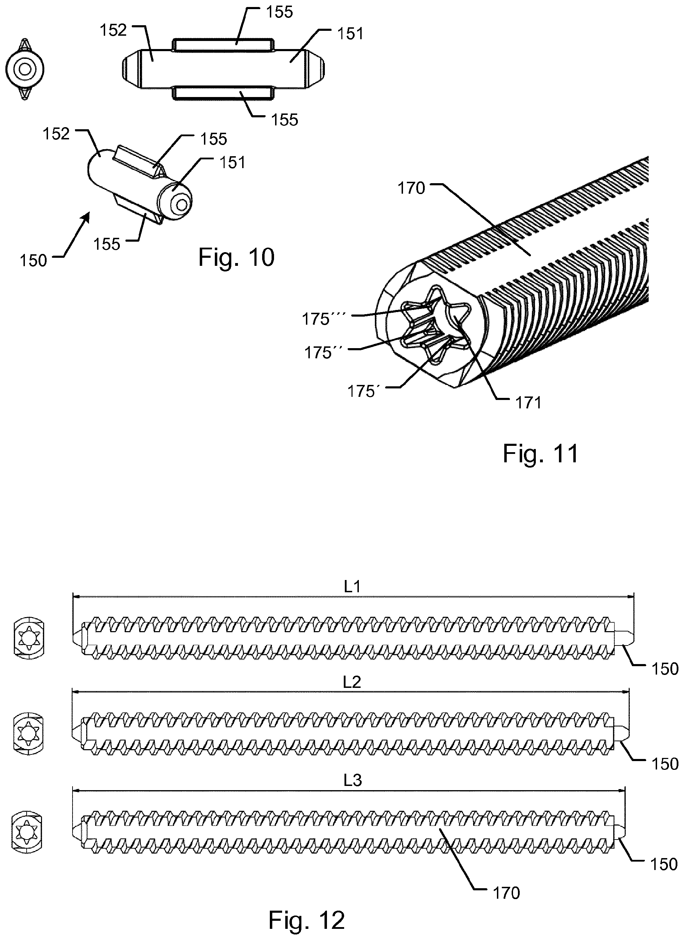

A third embodiment of a modified piston rod and adjustment member is shown in figs. 10-12. The third embodiment generally corresponds to the second embodiment shown in fig. 7-9 but compared to the second embodiment the adjustment member 150 of the third embodiment includes a second axially extending rib, so that two axially extending ribs 155 are arranged on opposite sides of the adjustment member 150 (see fig. 10). The piston rod 170 of the third embodiment is provided with a bore 171 having a contour system defining axial tracks in a star-shaped configuration leading into pairs of steps 175', 175" and 175''' arranged axially offset relative to the other pairs of the contour system. Each pair of steps includes oppositely positioned steps in a symmetrical configuration.

As shown in fig. 12, the third embodiment again provides variability of the assembly formed by the piston rod 170 and the adjustment member 150 can be chosen as L1, L2 or L3.

As described in connection with the first embodiment, any of the second and third embodiments may further be designed for the adjustment member 150 to be selectively insertable in the bore 171 of piston rod 170 in either of two ways providing a further means of varying the axial length of the assembly formed by the piston rod and the adjustment member. In this way the axial distance from the one or more radial extending protrusions to the end portions of the adjustment member may be designed in accordance with the desired variability. In further embodiments, the contour system 175 of the piston rod 170 forms a first contour system whereas the adjustment member 150 may be provided with a second contour system providing a similar arrangement of steps wherein each step is arranged axially offset with respect to the other steps. In such embodiment, the first contour system and the second contour system are configured to cooperate with each other to provide the desired variability.

In some embodiments the shape of the adjustment member is formed so as to enable insertion of the adjustment member into the bore 171 of the piston rod 170 selectively in two ways, either with the first end 151 or with the second end 152 pushed into the bore 171. If the adjustment member is formed with a protrusion where the distance L' is different from L", this may be utilized to create further variability of the tolerance compensation by appropriately choosing which end 151 or 152 that is to be selected for insertion into the bore 171.

In some embodiments, after the adjustment member has been inserted into the correct pocket and the axial blocking surfaces of the protrusion 155 abuts a selected step or a set of steps 175'/175'', the adjustment member is withheld in the bore 171, e.g. by means of a press-fitting or a snap-fitting. Alternatively, the adjustment member and the piston rod may be adhered or welded relative to each other, or secured mechanically by an alternative method.

Figs. 13-15 show a fourth embodiment whereas fig. 16 shows a fifth embodiment, which both do not form part of the invention.

In fig. 13 an adjustment member 250 is formed as a cylindrical object like the second embodiment but omitting the axial extending rib. In the shown embodiment, along a substantial part of the outer cylindrical surface the adjustment member 250 is formed with regularly arranged small circumferentially extending ribs.

The piston rod 270 of the fourth embodiment again is formed with a bore 271 that emerges from the distal facing surface of the piston rod. In the shown embodiment, the inner surface of the bore is along its length provided with regularly arranged small circumferentially extending ribs. The inner diameter of the bore 271 is formed slightly smaller than the outer diameter of the adjustment member 250 so that the adjustment member 250 is frictionally held by the bore 271 when the adjustment member is arranged in an initial axial position within bore 271. In the initial axial position, the adjustment member 250 extends distally from the distal end portion of the piston rod 270 by an extent larger than the clearance between the adjustment member and the piston. In accordance with the fourth embodiment at least one of the adjustment member and the piston rod includes at least one metallic portion arranged in the vicinity of the interface between the piston rod 270 and the adjustment member 250. In the shown embodiment the adjustment member 250 is formed by an inductively heatable metal.

During assembly operations wherein the cartridge and the piston rod are moved gradually closer to each other, the piston of the cartridge will push the adjustment member 250 further into the bore 271 until the cartridge and the piston rod reach their final destination relative to each other. By applying a suitable inductive field directed onto the metallic portion of the assembly made up by the piston rod and the adjustment member the surrounding material of the piston rod and/or the adjustment member will melt and provide a full adherence between the piston rod and the adjustment member. The adjustment member 250 is thus effectively secured to the piston rod 270 in the correct position to eliminate the clearance between the piston rod and the piston.

In alternative embodiments, during assembly, instead of moving the adjustment member 250 relative to the piston rod 270 by means of the piston of a cartridge, a separate tool may control the depth of insertion in accordance with a target position determined by means of measuring the axial position of the piston of the cartridge and/or the axial position of the piston rod relative to a reference point, such as a reference point on a housing component.

In the fifth embodiment shown in fig. 16, the adjustment member 250 is arranged within a through-going bore of the piston rod 270. In the shown embodiment, the adjustment member 250 is longer than the piston rod 270, and in the arrangement shown in fig. 16 the proximal portion of the adjustment member extends further proximal than the proximal end of the piston rod. However, it is to be noted that the adjustment member need not necessarily extend all the way to the proximal end of the piston rod provided a suitable tool is insertable into the through-going bore of the piston rod to operate the adjustment member. In such embodiment that is suitable in applications where the design of the drug delivery device, during assembly operations, enable manipulation of the adjustment member from locations near the proximal end of the piston rod 270 the adjustment member may be operated by means of a tool and moved relative to the piston rod in axial direction.

In the fifth embodiment, the through-going bore enables the adjustment member 250 to be moved in the distal direction relative to the piston rod 270. In applications where the cartridge and the piston rod assume their target assembly positions leaving a gap between the piston rod and the piston, the adjustment member 250 may be moved from an initial position within the piston rod towards a final position in engagement with the piston of the held cartridge, optionally by means of a cooperating piston washer intermediately arranged between the adjustment member and the piston.

In some embodiments of the above described configurations the end of the adjustment member 150 or 250 being intended to cooperate with the piston of a held cartridge may be so formed that the adjustment member exhibits a geometry forming a disc shape so as to act as a piston washer. Such disc shaped member may be provided freely rotatable relative to the remainder of the adjustment member or may be fixedly fitted not providing independent rotation. However, in the shown embodiments a single end or both ends of the adjustment member is formed with a pointed tip which is formed to cooperate with a bearing surface of a piston washer thus enabling the piston rod to rotate freely relative to the piston washer.

The injection device shown in fig. 1 provides a non-limiting example of a manual pen injector where the expelling mechanism is fully manual in which case the dose setting element and the injection button moves proximally during dose setting corresponding to the set dose size, and then is moved distally by the user to expel the set dose, this corresponding to the shown embodiments.

In alternative embodiments of injection devices these may incorporate an energy storage aiding to expel a set dose when a user operates a dose injection button. Depending on the type of expelling mechanism embodied in a given drug delivery device, the expelling mechanism may comprise a spring which is strained during dose setting and then released to drive the piston rod when the release button is actuated. Still other alternative mechanisms may include a spring member which stores sufficient energy for expelling the total contents of a cartridge during one or more separate dose administrations. Each such type of injection devices can be structured to enable assembly by the above described methods and features in accordance with the different aspects of the present invention.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the invention.

## Claims

1. A drug delivery device for dispensing one or more doses of a medicament, comprising:
- a medicament cartridge, wherein the cartridge comprises a body extending along an axis and defining a distal end and a proximal end, and a piston arranged slideably relative to the cartridge body,
- a drive mechanism comprising a piston rod (170) configured for axially moving the piston in the distal direction,
wherein the piston rod (170) connects to an adjustment member (150) located at least partially between the piston rod and a proximal face of the piston, wherein the adjustment member (150) is configured to abut or cooperate with the proximal face of the piston to reduce an axial clearance between the piston rod (170) and the proximal face of the piston, and wherein the relative rotational position between the piston rod (170) and the adjustment member (150) determines the axial position of the adjustment member (150) relative to the piston rod (170),
**characterized in that** one of the piston rod (170) and the adjustment member (150) comprises a first contour system (175) comprising a plurality of axially facing steps being circumferentially disposed and arranged axially offset from one another,
wherein the other of the piston rod (170) and the adjustment member (150) comprises at least one radially disposed protrusion (155),
wherein said at least one radially disposed protrusion (155) axially engages a selective one of said plurality of axially facing steps (175', 175", 175''') of the first contour system (175) so that the relative rotational position between the piston rod (170) and the adjustment member (150) determines the axial position of the adjustment member (150) relative to the piston rod (170) when the adjustment member (150) is positioned in full axial engagement with the piston rod (170), and
wherein the piston rod (170) comprises a first rotational block geometry and the adjustment member (150) comprises a second rotational block geometry to cooperate with the first rotational block geometry to prevent the said at least one radially disposed protrusion (155) from disengaging the selected one of said plurality of axially facing steps (175', 175", 175''') of the first contour system (175) when the adjustment member (150) is situated in full axial engagement with the piston rod (170).

2. A drug delivery device as defined in claim 1, wherein said at least one radially disposed protrusion (155) defines a plurality of radially disposed protrusions (155).

3. A drug delivery device as defined in claim 2, wherein the plurality of radially disposed protrusions (155) define a second contour system wherein the plurality of radially disposed protrusions (155) are arranged axially offset to one another to cooperate and engage with respective axially facing steps (175', 175'', 175''') of the first contour system (175).

4. A drug delivery device as defined in any of the claims 1-3, wherein each said plurality of axially facing steps (175', 175'', 175''') defines a bottom surface of a respective axially extending channel.

5. A drug delivery device as defined in any of the claims 1-4, wherein each of the at least one radially disposed protrusion (155) defines an abutment surface having a shape mating with an abutment surface defined by each of the plurality of axially facing steps (175', 175'', 175''').

6. A drug delivery device as defined in any of the claims 1-5, wherein said plurality of axially facing steps (175', 175'', 175''') each comprises a surface having a surface normal oriented substantially parallel with the axis.

7. A drug delivery device as defined in any of the claims 1-6, wherein, when the at least one radially disposed protrusion (155) engages a respective one of said series of axially facing steps (175', 175'', 175''') of the first contour system (175), a press-fit engagement between the piston rod (170) and the adjustment member (150) is provided.

8. A drug delivery device as defined in any of the claims 1-7, wherein one of the piston rod (170) and the adjustment member (150) defines an axially extending shaft member (151, 152) being received in an axial bore of the other of the piston rod (170) and the adjustment member (150).

9. A drug delivery device as defined in claim 8, wherein said axial bore is defined by an end portion having walls at least partly circumscribing the axial bore, and wherein the walls circumscribing the axial bore provides radial resiliency retaining said axially extending shaft member.

10. A drug delivery device as defined in any of the claims 1-9, wherein the adjustment member (150) defines said at least one radially disposed protrusion (155) and wherein the adjustment member (150) defines a first end and an opposite second end.

11. A drug delivery device as defined in claim 10, wherein the adjustment member (150) is configured for being inserted between the piston and the piston rod (170) selectively with the first end of the adjustment member (150) facing the piston rod (170) or with the second end of the adjustment member (150) facing the piston rod (170).

12. A drug delivery device as defined in claim 11, wherein respective ones of said at least one radially disposed protrusion (155) are arranged at an axial distance from the first end of the adjustment member (150) thereby defining a first set of respective distances and wherein respective ones of said at least one radially disposed protrusion (155) are arranged at an axial distance from the second end of the adjustment member (150) thereby defining a second set of respective distances and wherein the first set of respective distances differs from the second set of respective distances.

13. A drug delivery device as defined in claim 12, wherein a piston washer (9) is arranged between the adjustment member (150) and the piston and wherein the first end of the adjustment member (15) and/or the second end of the adjustment member (150) is formed with a rotation symmetrical portion configured for mating with a bearing surface of the piston washer (9).

14. A drug delivery device as defined in any of the claims 1-12, wherein a piston washer (9) is arranged between the adjustment member (150) and the piston.

15. A drug delivery device as defined in any of the claims 1-14, wherein the drug delivery device forms a disposable device wherein the cartridge mounted within the device cannot be replaced with a new cartridge.

## Patentansprüche

1. Wirkstofffreisetzungsvorrichtung zur Abgabe einer oder mehrerer Dosen eines Medikaments, umfassen:
- eine Medikamentenkartusche, wobei die Kartusche einen Körper umfasst, der sich entlang einer Achse erstreckt und ein distales Ende und ein proximales Ende definiert, und einen Kolben, der verschiebbar in Bezug auf den Kartuschenkörper angeordnet ist,
- einen Antriebsmechanismus, der Kolbenstange (170) umfasst, die für das axiale Bewegen des Kolbens in die distale Richtung konfiguriert ist,
wobei Kolbenstange (170) mit Anpassungselement (150) verbunden ist, das sich zumindest teilweise zwischen der Kolbenstange und einer proximalen Fläche des Kolbens befindet, wobei Anpassungselement (150) so konfiguriert ist, dass es an der proximalen Fläche des Kolbens anliegt oder mit dieser zusammenwirkt, um einen axialen Zwischenraum zwischen Kolbenstange (170) und der proximalen Fläche des Kolbens zu verringern, und wobei die relative Drehposition zwischen Kolbenstange (170) und Anpassungselement (150) die axiale Position von Anpassungselement (150) in Bezug auf Kolbenstange (170) bestimmt,
**dadurch gekennzeichnet, dass** eines von Kolbenstange (170) und Anpassungselement (150) erstes Umrisssystem (175) umfasst, das eine Vielzahl von axial gerichteten Stufen umfasst, die am Umfang angeordnet und axial versetzt voneinander vorgesehen sind, wobei das andere von Kolbenstange (170) und Anpassungselement (150) mindestens einen radial angeordneten Vorsprung (155) umfasst,
wobei besagter mindestens eine radial angeordneter Vorsprung (155) mit einer entsprechenden der Vielzahl von axial gerichteten Stufen (175', 175'', 175''') von erstem Umrisssystem (175) im Eingriff befindet, sodass die relative Drehposition zwischen Kolbenstange (170) und Anpassungselement (150) die axiale Position von Anpassungselement (150) in Bezug auf Kolbenstange (170) bestimmt, wenn Anpassungselement (150) im vollen Eingriff mit Kolbenstange (170) positioniert ist, und wobei Kolbenstange (170) eine erste Drehblockgeometrie umfasst und Anpassungselement (150) eine zweite Drehblockgeometrie umfasst, um mit erster Drehblockgeometrie zusammenzuwirken, um zu verhindern, dass besagter mindestens eine radial angeordneter Vorsprung (155) von den ausgewählten besagten der Vielzahl von axial gerichteten Stufen (175', 175'', 175''') von erstem Umrisssystem (175) nicht mehr im Eingriff steht, wenn Anpassungselement (150) sich im vollständigen axialen Eingriff mit Kolbenstange (170) befindet.

2. Wirkstofffreisetzungsvorrichtung, wie in Anspruch 1 definiert, wobei besagter mindestens eine radial angeordneter Vorsprung (155) eine Vielzahl von radial angeordneten Vorsprüngen (155) definiert.

3. Wirkstofffreisetzungsvorrichtung, wie in Anspruch 2 definiert, wobei die Vielzahl von radial angeordneten Vorsprüngen (155) ein zweites Umrisssystem definieren, wobei die Vielzahl von radial angeordneten Vorsprüngen (155) axial versetzt zueinander angeordnet sind, um mit jeweiligen axial gerichteten Stufen (175', 175", 175''') von erstem Umrisssystems (175) zusammenzuwirken und im Eingriff zu stehen.

4. Wirkstofffreisetzungsvorrichtung, wie in einem der Ansprüche 1 bis 3 definiert, wobei jede der besagten Vielzahl von axial ausgerichteten Stufen (175', 175", 175''') eine untere Fläche eines jeweiligen axial verlaufenden Kanals definiert.

5. Wirkstofffreisetzungsvorrichtung, wie in einem der Ansprüche 1 bis 4 definiert, wobei jeder der mindestens einen radial angeordneten Vorsprünge (155) eine Anlagefläche mit einer Form definiert, die mit einer Anlagefläche zusammenpasst, die durch jede der Vielzahl von axial gerichteten Stufen (175', 175'', 175''') definiert wird.

6. Wirkstofffreisetzungsvorrichtung, wie in einem der Ansprüche 1 bis 5 definiert, wobei jede der besagten Vielzahl von axial gerichteten Stufen (175', 175'', 175''') jeweils eine Fläche umfasst, die eine Flächen-Normale aufweist, die im Wesentlichen parallel zur Achse ausgerichtet ist.

7. Wirkstofffreisetzungsvorrichtung, wie in einem der Ansprüche 1 bis 6 definiert, wobei, wenn mindestens ein radial angeordneter Vorsprung (155) mit einer jeweiligen der besagten Reihe axial gerichteter Stufen (175', 175", 175''') des ersten Umrisssystems (175) im Eingriff steht, ein Presspassungseingriff zwischen der Kolbenstange (170) und dem Anpassungselement (150) bereitgestellt wird.

8. Wirkstofffreisetzungsvorrichtung, wie in einem der Ansprüche 1 bis 7 definiert, wobei eines von Kolbenstange (170) und Anpassungselement (150) ein sich axial erstreckendes Schaftelement (151, 152) definiert, das in einer axialen Bohrung des anderen von Kolbentange (170) und Anpassungselement (150) aufgenommen wird.

9. Wirkstofffreisetzungsvorrichtung, wie in Anspruch 8 definiert, wobei besagte axiale Bohrung durch einen Endabschnitt definiert ist, der Wände aufweist, die zumindest teilweise die axiale Bohrung umgeben, und wobei die Wände, die die axiale Bohrung umgeben, radiale Elastizität zum Halten des sich axial erstreckenden Schaftelements bereitstellen.

10. Wirkstofffreisetzungsvorrichtung, wie in einem der Ansprüche 1 bis 9 definiert, wobei Anpassungselement (150) besagten mindestens einen radial angeordneten Vorsprung (155) definiert und wobei Anpassungselement (150) ein erstes Ende und ein gegenüberliegendes zweites Ende definiert.

11. Wirkstofffreisetzungsvorrichtung, wie in Anspruch 10 definiert, wobei Anpassungselement (150) für das selektive Einstecken zwischen Kolben und Kolbenstange (170) mit erstem Ende von Anpassungselement (150) zur Kolbenstange (170) gerichtet oder mit zweitem Ende von Anpassungselement (150) zur Kolbenstange (170) gerichtet konfiguriert ist.

12. Wirkstofffreisetzungsvorrichtung, wie in Anspruch 11 definiert, wobei jeweilige besagten des mindestens einen radial angeordneten Vorsprungs (155) in einem axialen Abstand vom ersten Ende von Anpassungselement (150) angeordnet sind und dadurch einen ersten Satz von jeweiligen Abständen definieren, und wobei jeweilige des besagten mindestens einen radial angeordneten Vorsprungs (155) in einem axialen Abstand vom zweiten Ende des Anpassungselement (150) angeordnet sind und dadurch einen zweiten Satz von jeweiligen Abständen definieren, und wobei erster Satz von jeweiligen Abständen sich vom zweiten Satz von jeweiligen Abständen unterscheidet.

13. Wirkstofffreisetzungsvorrichtung, wie in Anspruch 12 definiert, wobei Kolbenscheibe (9) zwischen Anpassungselement (150) und dem Kolben angeordnet ist und wobei erstes Ende von Anpassungselement (15) und/oder zweites Ende von Anpassungselement (150) mit einem rotationssymmetrischen Abschnitt ausgebildet ist, der so konfiguriert ist, dass dieser mit einer Auflagefläche von Kolbenscheibe (9) zusammenpasst.

14. Wirkstofffreisetzungsvorrichtung, wie in einem der Ansprüche 1 bis 12 definiert, wobei Kolbenscheibe (9) zwischen Anpassungselement (150) und dem Kolben angeordnet ist.

15. Wirkstofffreisetzungsvorrichtung, wie in einem der Ansprüche 1 bis 14 definiert, wobei die Wirkstofffreisetzungsvorrichtung eine Einweg-Vorrichtung ausbildet, wobei die in der Vorrichtung montierte Kartusche nicht durch eine neue Kartusche ersetzt werden kann.

## Revendications

1. Dispositif de délivrance de médicaments pour l'administration d'une ou plusieurs doses d'un médicament, comprenant :
- une cartouche de médicament, dans laquelle la cartouche comprend un corps s'étendant le long d'un axe et définissant une extrémité distale et une extrémité proximale, et un piston agencé de manière coulissante par rapport au corps de la cartouche,
- un mécanisme d'entraînement comprenant une tige de piston (170) configurée pour le déplacement axial du piston dans la direction distale,
dans lequel la tige de piston (170) se connecte à un élément de réglage (150) situé au moins partiellement entre la tige de piston et une face proximale du piston, dans lequel l'élément de réglage (150) est configuré pour venir en butée ou coopérer avec la face proximale du piston pour réduire un jeu axial entre la tige de piston (170) et la face proximale du piston, et dans lequel la position de rotation relative entre la tige de piston (170) et l'élément de réglage (150) détermine la position axiale de l'élément de réglage ( 150) par rapport à la tige de piston (170),
**caractérisé en ce que** une de la tige de piston (170) et de l'élément de réglage (150) comprend un premier système de contour (175) comprenant une pluralité de crans se faisant face axialement étant disposés circonférentiellement et agencés en décalage axial l'un par rapport à l'autre,
dans lequel l'autre de la tige de piston (170) et de l'élément de réglage (150) comprend au moins une protrusion disposée radialement (155),
dans lequel ladite au moins une protrusion disposée radialement (155) engage axialement une partie de ladite pluralité de crans se faisant face axialement (175', 175'', 175''') du premier système de contour (175) afin que la position relative de rotation entre la tige de piston (170) et l'élément de réglage (150) détermine la position axiale de l'élément de réglage (150) par rapport à la tige du piston (170) lorsque l'élément de réglage (150) est positionné en engagement axial complet avec la tige de piston (170), et
dans lequel la tige de piston (170) comprend une première géométrie de bloc de rotation et l'élément de réglage (150) comprend une deuxième géométrie de bloc de rotation pour coopérer avec la première géométrie de bloc de rotation pour empêcher ladite au moins une protrusion disposée radialement (155) d'un désengagement du sélectionné parmi ladite pluralité de crans se faisant face axialement (175', 175'' 175''') du premier système de contour (175) lorsque l'élément de réglage (150) est situé en engagement axial complet avec la tige de piston (170).

2. Dispositif de délivrance de médicament selon la revendication 1, dans lequel ladite au moins une protrusion disposée radialement (155) définit une pluralité de protrusions disposées radialement (155).

3. Dispositif de délivrance de médicament selon la revendication 2, dans lequel la pluralité de protrusions disposées radialement (155) définit un deuxième système de contour dans lequel la pluralité de protrusions disposées radialement (155) sont agencées axialement en décalage l'une par rapport à l'autre pour coopérer et s'engager avec des crans respectifs se faisant face axialement (175', 175", 175''') du premier système de contour (175).

4. Dispositif de délivrance de médicament selon l'une quelconque des revendications 1-3, dans lequel chaque pluralité de crans se faisant face axialement (175', 175", 175'''') définit une surface inférieure d'un canal d'extension axial respectif.

5. Dispositif de délivrance de médicament tel que défini dans l'une quelconque des revendications 1-4, dans lequel chacun de l'au moins une protrusion disposée radialement (155) définit une surface de butée ayant une forme de contact avec une surface de butée définie par chacune de la pluralité de crans se faisant face axialement (175', 175", 175''').

6. Dispositif de délivrance de médicament selon l'une quelconque des revendications 1-5, dans lequel ladite pluralité de crans se faisant face axialement (175', 175", 175") comprend chacune une surface ayant une surface normale orientée substantiellement parallèlement à l'axe.

7. Dispositif de délivrance de médicament selon l'une quelconque des revendications 1-6, dans lequel, lorsque l'au moins une protrusion disposée radialement (155) engage une série respective de ladite série de crans se faisant face face axialement (175', 175'', 175''') du premier système de contour (175), un engagement par ajustement serré entre la tige du piston (170) et l'élément de réglage (150) est fourni.

8. Dispositif de délivrance de médicament tel que défini dans l'une quelconque des revendications 1-7, dans lequel une de la tige de piston (170) et de l'élément de réglage (150) définit un élément d'arbre s'étendant axialement (151, 152) étant reçu dans un alésage axial de l'autre de la tige du piston (170) et de l'élément de réglage (150).

9. Dispositif de délivrance de médicament selon la revendication 8, dans lequel ledit alésage axial est défini par une partie d'extrémité ayant des parois circonscrivant au moins partiellement l'alésage axial, et dans lequel les parois circonscrivant l'alésage axial fournissent une élasticité radiale retenant ledit élément d'arbre s'étendant axialement.

10. Dispositif de délivrance de médicament selon l'une quelconque des revendications 1-9, dans lequel l'élément de réglage (150) définit ladite au moins une protrusion disposée radialement (155) et dans lequel l'élément de réglage (150) définit une première extrémité et une deuxième extrémité opposée.

11. Dispositif de délivrance de médicament selon la revendication 10, dans lequel l'élément de réglage (150) est configuré pour être inséré entre le piston et la tige du piston (170) de manière sélective avec la première extrémité de l'élément de réglage (150) faisant face à la tige du piston (170) ou avec la deuxième extrémité de l'élément de réglage (150) faisant face à la tige du piston (170).

12. Dispositif de délivrance de médicament selon la revendication 11, dans lequel celles respectives de ladite au moins une protrusion disposée radialement (155) sont disposées à une distance axiale de la première extrémité de l'élément de réglage (150) définissant ainsi un premier ensemble de distances respectives et dans lequel celles respectives de ladite au moins une protrusion disposée radialement (155) sont agencées à une distance axiale de la deuxième extrémité de l'élément de réglage (150) définissant ainsi un deuxième ensemble de distances respectives et dans lequel le premier ensemble de distances respectives diffère du deuxième ensemble de distances respectives.

13. Dispositif de délivrance de médicament selon la revendication 12, dans lequel une rondelle de piston (9) est disposée entre l'élément de réglage (150) et le piston, et dans lequel la première extrémité de l'élément de réglage (15) et/ ou la deuxième extrémité de l'élément de réglage (150) est formée avec une partie symétrique de rotation configurée pour un contact avec une surface de roulement de la rondelle du piston (9).

14. Dispositif de délivrance de médicament selon l'une quelconque des revendications 1-12, dans lequel une rondelle de piston (9) est agencée entre l'élément de réglage (150) et le piston.

15. Dispositif de délivrance de médicament selon l'une quelconque des revendications 1-14, dans lequel le dispositif de délivrance de médicament forme un dispositif jetable dans lequel la cartouche montée dans le dispositif ne peut pas être remplacée par une nouvelle cartouche.
